# EUROPEAN PATENT SPECIFICATION

(11) **EP 2 780 051 B1**
(45) Date of publication and mention of the grant of the patent: **24.10.2018**
(21) Application number: 12847942.5
(22) Date of filing: 08.11.2012
(51) Int. Cl.: A61L 29/08, A61L 29/16, A61L 29/10, A61M 25/01

(54) **NOVEL ENHANCED FORMULATIONS FOR COATING MEDICAL DEVICES**
NEUE VERBESSERTE FORMULIERUNGEN ZUR BESCHICHTUNG MEDIZINISCHER VORRICHTUNGEN
NOUVELLES FORMULATIONS AMÉLIORÉES DE REVÊTEMENT DE DISPOSITIFS MÉDICAUX

(30) Priority: 09.11.2011 US 201113292636; 01.03.2012 US 201261605590 P
(43) Date of publication of application: 24.09.2014
(73) Proprietor: Arrow International, Inc., Reading, PA 19605 (US)
(72) Inventor: GIARE-PATEL, Kamna, Wyomissing, Pennsylvania 19610 (US); GUPTA, Nisha, Reading, Pennsylvania 19605 (US); ETTER, Greg, Reading, Pennsylvania 19605 (US); SECHRIST, Kevin, Reading, Pennsylvania 19605 (US); STEWART, Molly, Reading, Pennsylvania 19605 (US); TENTLER, Igor, Reading, Pennsylvania 19605 (US); WILLIAMS, Al, Pennsylvania 19605 (US)
(74) Representative: CMS Cameron McKenna Nabarro Olswang LLP
(86) International application number: PCT/US2012/064203
(87) International publication number: WO 2013/070951

(56) References cited:
- WO-A2-02/34311
- KR-A- 20110 100 247
- US-A- 5 942 239
- US-A1- 2004 052 831
- US-A1- 2004 208 908
- US-A1- 2007 048 345
- US-B2- 6 872 195

## Description

### Field of the disclosure

The present disclosure relates in particular to a coated or impregnated medical device with an antimicrobially effective amount of chlorhexidine, and to methods for coating or impregnating a medical device with an antimicrobially effective amount of chlorhexidine.

### Background of the disclosure

Catheters and other devices that are implanted into vessels or cavities in the clinical or veterinary situation are associated with infections, including biofilms on the medical device, local infections, and bloodstream infections. Catheter-related bloodstream infections affect over 2 million hospitalized patients per year (Krein, et al (2007) Mayo Clin. Proc. 82:672-678). Certain catheters are accessed multiple times per day, for example, for taking measurements or obtaining samples for laboratory analysis. Multiple samplings increase the potential for contamination and infections. Short-term catheters are more associated with microbial contamination of the external surface of the catheter, while internal surface or lumenal microbial colonization is associated with long-term implantation. Catheters, catheter cuffs, and other medical devices are sometimes coated or impregnated with antimicrobial or antiseptic agents, with the goal of decreasing infections. Use of catheters impregnated with agents, such as chlorhexidine, can partially reduce the risk of infections (see, e.g., Trautner and Darouiche (2004) Arch. Intern. Med. 164:842-850). Chlorhexidine has been used for coating medical devices, including catheters, cuffs, and synthetic membranes (see, e.g., O'Grady, et al (2002) Pediatrics 1 10:e51 -e75; Chen, et al (2003) J. Periodontol. 74:1652-1659). This agent has broad activity against gram positive and negative bacteria, as well as against yeasts and some viruses (Milstone, et al. (2008) Healthcare Epidemiology 46:274-281).
WO 02/34311 describes techniques for applying coatings of different substances onto different portions of the surface of an implantable device, for example coating stents. The coating substance may include polymers loaded with a therapeutic substance and that the substance may include a solvent.
US 6872195 B2 describes polymeric medical articles comprising the anti-infective agents chlorhexidine and triclosan. A hydrophobic polymeric medical article is treated by dipping the article in a treatment solution of hydrophilic polymer comprising these compounds. The additional use of a silver salt (such as silver sulfadiazine) is also disclosed.

### Summary of the disclosure

The scope of this invention is defined by the claims. Any references in the description to methods of treatment refer to the compositions and medical devices of the present invention for use in a method for treatment of the human (or animal) body by therapy.

There is provided a medical device according to claim 1 and a method for coating or impregnating a medical device according to claim 3. A selection of optional features is set out in the dependent claims.

The first formulation, as referred to herein, includes methyl-ethyl-keton 50-70%, methanol 10-20%, acetone 15-25%, chlorhexidine diacetate 0.5-4% and chlorhexidine free base 0.5-4%. The second formulation, as referred to herein, includes tetrahydrofuran 70-90% by weight, methanol 5-15%, polyurethane 1-15% and chlorhexidine diacetate 0.5-4%.

In medical device embodiments, what is provided is a medical device comprising an interior surface that defines a cavity or lumen, and an exterior surface, wherein the interior surface is treated with the first formulation as defined in the independent claims, resulting in coating or impregnation with an antimicrobially effective amount of chlorhexidine, wherein the exterior surface of the medical device is treated with the second formulation as defined in the independent claims, resulting in coating or impregnation with an antimicrobially effect amount of chlorhexidine. Also provided is above medical device, that comprises one or more of a catheter, cannula, introducer, dilator, or sheath. The above medical device does not comprise triclosan and does not comprise silver salt.

In methods embodiments, what is provided is a method for coating or impregnating a medical device, the medical device comprising an inside surface, and a cavity or lumen that is defined by said inside surface, wherein the medical device further comprises an outside surface or exterior surface, wherein the method comprises contacting a first formulation, as defined in the independent claims, to the inside surface, and contacting a second formulation, as defined in the independent claims, to the outside surface, and where the first and second formulations have a different composition from each other. The second formulation includes a dissolved polymer that comprises polyurethane.

Also provided is above method, comprising contacting of the first formulation to the inside surface resulting in the coating or impregnation to the inside surface of an anti-microbially effective amount of chlorhexidine, and comprising contacting of the second formulation to the outside surface resulting in the coating or impregnation to the outside surface of an anti-microbially effective amount of chlorhexidine.

In medical device embodiments, what is provided is a medical device prepared by any one of the above methods. Encompassed is medical device that comprises one or more of a catheter, cannula, introducer, dilator, or sheath. According to the claimed invention, the medical device does not comprise triclosan and does not comprise silver salt.

Methods of manufacturing are also described herein. What is embraced is a method for manufacturing the first formulation, comprising combining and mixing at least two of said methyl-ethyl-ketone, methanol, acetone, chlorhexidine diacetate, and chlorhexidine free base, wherein said combining and mixing completes the combining together of all of said methyl-ethyl-ketone, methanol, acetone, chlorhexidine diacetate, and chlorhexidine free base. What is embraced is a method for manufacturing the second formulation, comprising combining and mixing at least two of said tetrahydrofuran (THF), methanol, polyurethane, and chlorhexidine diacetate, wherein said combining and mixing completes the combining together of all of said tetrahydrofuran (THF), methanol, polyurethane, and chlorhexidine diacetate.

Embodiments that result in reduced thickening of intima are provided, including any one of the above medical devices that results in reduced intima thickening following dwelling in a vein, when compared to a control medical device. What is provided is above medical device, wherein the control device is treated with a formulation that does not contain chlorhexidine, or wherein the control device is not treated with any formulation. What is provided is any one of above medical devices, that does not further comprise an anti-thrombogenic agent, wherein in use and with continued residence in a subject for at least one week, thrombogenesis occurs at a reduced rate of thrombus formation, wherein the reduced rate is tested by comparing the rate (X thrombi/week) of thrombus formation associated with said medical device, with the rate (Y thrombi/week) of thrombus formation associated with a corresponding medical device that does is not coated or impregnated with chlorhexidine. What is provided is above medical device, wherein X is selected from one of less than 90% of Y, less than 80% of Y, and less than 70% of Y. What is provided is any one of above medical devices, that further comprises at least one anti-thrombogenic agent, wherein the at least one anti-thrombogenic agent is provided separately from the first formulation and second formulation. What is provided is any one of above medical devices, that configured to introduce fluids into a subject, to withdraw fluids from the subject, or to both introduce and withdraw fluids, wherein in operation the device is capable of dwelling in a physiological vessel or chamber, and is capable of introducing, withdrawing, or both introducing and withdrawing fluids to said physiological vessel or chamber, wherein in use the fluids are in contact with and transmitted by said cavity or lumen that is defined by said inside surface during the introducing and withdrawing. What is provided is above medical device, wherein the vessel is a vein.

Moreover, the disclosure provides the above method, wherein the medical device comprises a catheter or cannula. In device embodiment, what is provided is a medical device prepared by one or more of the above methods of, wherein the medical device comprises chlorhexidine, or comprises detectable chlorhexidine. In yet another methods embodiment, disclosure provides a method for using the above medical device, wherein in use, the medical device is inserted into a vascular lumen of a subject or patient, the medical device dwells in the vascular lumen for a period of at least ten seconds, and wherein the medical is removed from the vascular lumen. In exclusionary or negative embodiments, the disclosure provides any one of the above-disclosed medical devices, where the medical device does not comprise triclosan and does not comprise silver salt. In another aspect, the disclosure excludes formulation that comprises zinc acetate, excludes a formulation comprising zinc lactate, excludes a formulation comprising a water-soluble zinc salt, or excludes any combination of the above. In embodiments, the disclosure excludes a formulation that comprises panthenol, octoxyglycerin, phenoxyethanol, iodine compound, or parachlorometaxylenol, and that excludes any combination of the above. In other exclusionary embodiments, what is excluded is a formulation that comprises octoxyglycerin, miconazole, or the combination of octoxyglycerin and miconazole.

Device exclusionary embodiments encompass the following. Without implying any limitation to the present disclosure, device exclusionary embodiments can exclude a device coated with, or impregnated with zinc acetate, zinc lactate, a water-soluble zinc salt, panthenol, octoxyglycerin, phenoxyethanol, iodine compound, parachlorometaxylenol, octoxyglycerin, miconazole, combination of oxtoxyglycerin and miconazole, or any exclusionary combination of the above.

In time embodiments, method of treatment of medical device with formulation comprises contacting medical device with formulation for 30 seconds or less, 60 seconds or less, 2min or less, 4min or less, 6min or less, 8min or less, 10min or less, 15min or less, 20min or less, 30min or less, 40min or less, 50min or less, 60min or less, 2h or less, 3h or less, 4h or less, and the like. Other time embodiments include 30-60sec, 1min-2min, 2min-4min, 1min-4min, 1min-5min, 5min-10min, 5min-20min, 10min-60min, and the like. What is contemplated is contacting, treating, dipping, coating, impregnating, a time that ensures that an anti-microbially effective amount of anti-microbial agent is coated or impregnated, any combination thereof, and the like. In other time embodiments, external coating time is less than 10 seconds, less than 8sec, less than 6sec, less than 4sec, less than 3sec, less than 2sec, less than 1sec, less than 0.8sec, less than 0.6sec, less than 0.4sec, and so on, where a thin, uniform layer of solution is applied to the exterior, and immediately starts to dry. In embodiments, there is no true "immersion" during external coating. Timing of internal coating can be controlled by pressurized blow-out, to remove solvent from interior of medical device. Internal coating time is about 4 seconds, about 6sec, about 8sec, about 10sec, about 12sec, about 14sec, about 16sec, about 18sec, about 20sec, about 25sec, about 30sec, about 40sec, about 60sec, about 90sec, about 2min, about 4min, about 6min, about 8min, about 10min, and so on.

In soluble polymer embodiments, what is provided is a formulation containing about 0.2%, about 0.5%, about 1.0%, about 1.5%, about 2.0%, about 2.5%, about 3.0%, about 3.5%, about 4.0%, about 4.5%, about 5.0%, about 6.0%, about 7.0%, about 8.0%, about 9.0%, about 10%, and the like, of soluble polymer, such as soluble polyurethane. In other aspects, what is provided is a formulation with greater than 0.2%, 0.5%, 1.0%, 1.5%, 2.0%, 2.5%, 3.0%, 3.5%, 4.0%, 4.5%, 5.0%, 6.0%, 7.0%, 8.0%, 9.0%, greater than 10%, and the like, or lesser than 0.2%, 0.5%, 1.0%, 1.5%, 2.0%, 2.5%, 3.0%, 3.5%, 4.0%, 4.5%, 5.0%, 6.0%, 7.0%, 8.0%, 9.0%, lesser than 10%, and the like, of soluble polymer.

In methods embodiment, the present disclosure provides method for coating or impregnating a medical device, the medical device comprising an inside surface, and a cavity or lumen that is defined by said inside surface, wherein the medical device further comprises an outside surface or exterior surface, wherein the method comprises contacting a first formulation to the inside surface, and contacting a second formulation to the outside surface, and where the first and second formulations have a different composition from each other. In yet another methods embodiment, the disclosure provides the above method wherein the second formulation comprises a dissolved polymer, as well as the above method wherein the dissolved polymer of the second formulation comprises polyurethane, as well as the above method, wherein the first formulation is the above formulation (that does not include polyurethane), and the second formulation (the formulation including polyurethane). In yet another methods embodiment, the present disclosure provides the above method, wherein the first formulation comprises methyl-ethyl-ketone, methanol, and acetone, and under 10% tetrahydrofuran, and the second formulation comprises tetrahydrofuran, methanol, and a dissolved plastic polymer, and under 10% methyl-ethyl-ketone. In yet another methods embodiment, what is provided is the above method, comprising contacting of the above formulation (that does not include polyurethane) to the inside surface resulting in the coating or impregnation to the inside surface of an anti-microbially effective amount of chlorhexidine, and comprising contacting of the above formulation (that does include polyurethane) to the outside surface resulting in the coating or impregnation to the outside surface of an anti-microbially effective amount of chlorhexidine. In a device embodiment, the present disclosure provides a medical device provided by the above method, as well as a medical device that comprises a catheter, cannula, or introducer. The above medical device does not comprise triclosan and does not comprise silver salt.

What is also provided is the combination of medical device and a formulation, for example, combinations where medical device is being soaked in formulation, where medical device is being partially or fully submersed in formulation, or where medical device is being perfused with formulation. Present invention provides combination of a medical device with the formulation of one or both of the above formulations (the one not including polyurethane; the one including polyurethane).

In manufacturing embodiments, present disclosure includes a method for manufacturing the above formulation (the formulation not including polyurethane), comprising combining and mixing at least two of said methyl-ethyl-ketone, methanol, acetone, chlorhexidine diacetate, and chlorhexidine free base, wherein said combining and mixing completes the combining together of all of said methyl-ethyl-ketone, methanol, acetone, chlorhexidine diacetate, and chlorhexidine free base. In another manufacturing embodiment, what is provided is a method for manufacturing the above formulation (the formulation that includes polyurethane), comprising combining and mixing at least two of said tetrahydrofuran (THF), methanol, polyurethane, and chlorhexidine diacetate, wherein said combining and mixing completes the combining together of all of said tetrahydrofuran (THF), methanol, polyurethane, and chlorhexidine diacetate. In another manufacturing embodiment, what is provided is a method for coating or impregnating a medical device with chlorhexidine, wherein the medical device comprises an interior surface and exterior surface, comprising contacting the interior surface with the above formulation (not including polyurethane), resulting in coating the interior surface with an anti-microbially effective amount of chlorhexidine, or contacting the exterior surface with the above formulation (the formulation that does include polyurethane), resulting in coating the exterior surface with an anti-microbially effective amount of chlorhexidine, or contacting both the interior surface with the above formulation of (not including polyurethane) and the exterior surface with the above formulation (formulation that does include polyurethane), resulting in resulting in coating the interior surface and the exterior surface with an anti-microbially effective amount of chlorhexidine.

"Coating" encompasses, and is not limited to, impacting to at least a surface of a device at least one of antimicrobials and anti-thrombogenic agents, and the objects of such. A coating can include, without limitation, an agent that is embedded within the coating, an agent that is surface-associated to the coating's exterior, an agent that is covalently linked to the coating (to interior, to exterior, or to both aspects of the coating), that is non-covalently linked to the coating (to interior, to exterior, or to both aspects of the coating), and any combination thereof. The agent can be, for example, chlorhexidine.

Anti-thrombogenic agent can be one or more of, for example, heparin, urokinase, streptokinase, Warfarin, dicoumarol, tissue plasminogen activator (TPA). Although chlorhexidine is not an "anti-thrombogenic agent," or is not classified as an "anti-thrombogenic agent," the present disclosure provides medical devices coated or impregnated with chlorhexidine, where an effect of this chlorhexidine is that of anti-thrombogenicity.

What is provided is a medical device where one or more anti-thrombogenic agents is provided by a first formulation, by a second formulation, by both a first formulation and a second formulation, or by way of a formulation that is not the first or second formulation.

The skilled artisan will understand that the antimicrobial agent of the present disclosure prevents or reduced microbial growth on a medical device, such as a catheter, dilator, sheath, valve. The skilled artisan will understand that use of an agent to reduce growth of bacteria, fungi, or other microbes on a medical device does not constitute a method of medical treatment. The skilled artisan will also understand that anti-thrombogenic agent of the present disclosure concerns an interaction between a medical device and one or more enzymes or proteins, and that this is not a method of medical treatment.

What is embraced by a formulation for external application or soaking that comprises a dissolved plastic polymer. The dissolved plastic polymer can be more or more of, or any combination of, polyurethane, polyethylene, polyethlyene teraphthalate, ethylene vinyl acetate, silicone, tetrafluoroethylene, polypropylene, polyethylene oxide, polyacrylate, and so on. What is encompassed are coatings, coating solutions, and medical devices that are coated with coating solutions, using Carbothane® family of polycarbonate-based aliphatic and aromatic polyurethanes, Estane®, which is a thermoplastic polyurethane, Pellethane®, which is a family of medical-grade polyurethane elastomers and exceptionally smooth surfaces, Tecoflex®, which is a family of aliphatic polyether polyurethanes, where low durometer versions are particularly suitable for long-term implant applications, Tecothane®, an aromatic polyurethane, Texin®, an aromatic polyether-based polyurethane which allows for very thin gauges (Microspec Corp., Peterborough, NH; Lubrizol, Inc., Wickliffe, Ohio; Entec Polymers, Orlando, FL). See, U.S. Pat. Nos. 6,565,591 of Brady, 7,029,467 of Currier, and 7,892,469 of Lim. In embodiments, the present disclosure provides the recited polymers for use in coating solutions, or for use in manufacturing the medical device that is to be coated. In exclusionary embodiments, what is provided is a formulation for coating, or a medical device coated with said coating, where the only polymer in the coating is Tecoflex, Texothane, Texin, Carbothane, Estane, or Pellethane. For example, what is provided is a formulation that does not include Pellethane.

In embodiments where an interior is treated with a first formulation (A) and an exterior is treated with a second formulation (B), contact of the interior by the first formulation (A) and contact of the same interior by the second formulation (B) occurs, in some embodiments, at a ratio of greater than (A)/(B)=80/20, greater than (A)/(B)=85/15, greater than (A)/(B)=90/10, greater than (A)/(B)=95/5, greater than (A)/(B)=98/2, greater than (A)/(B)=99/1 , greater than (A)/(B)=99.9/0.1, and so on. What is also contemplated, are embodiments where an exterior is treated with a first formulation (C) and an interior is treated with a second formulation (D), contact of the exterior by the first formulation (C) and contact of the same exterior by the second formulation (D) occurs, in certain embodiments, at a ratio of greater than (C)/(D)=80/20, greater than (C)/(D)=85/15, greater than (C)/(D)=90/10, greater than (C)/(D)=95/5, greater than (C)/(D)=98/2, greater than (C)/(D)=99/1, greater than (C)/(D)=99.9/0.1, and so on.

### Brief descriptions of the figures

Figure 1. Cumulative elution of chlorhexidine (micrograms/cm) over time, where treatment of catheters was with 0.5% or 1 .5% chlorhexidine.
Figure 2. Cumulative elution of chlorhexidine (percent release) over time, where treatment of catheters was with 0.5% or 1 .5% chlorhexidine.
Figure 3. Cumulative elution of chlorhexidine (micrograms/cm), where treatment of catheters was with 1 .5% or 3.0% chlorhexidine.
Figure 4. Cumulative elution of chlorhexidine (percent release) over time, where treatment of catheters was with 1 .5% or 3.0% chlorhexidine.
Figure 5. Quantity of chlorhexidine eluted per day.
Figure 6. Cumulative elution of chlorhexidine.
Figure 7. Burst pressure (psi) of treated catheters.
Figure 8. Fibrin sheath weight as percent of control (non-infection model).
Figure 9. Fibrin sheath weight as percent of control (infection model).
Figure 10. Intimal hyperplasia as percentage of vein diameter.

### Detailed description of the disclosure

The present disclosure provides formulations, as well as medical devices treated with or impregnated with, the formulations of the present disclosure. Catheters and other medical devices, treated or impregnated with an antimicrobial agent, and configured for use in different regions of the body, are provided. These include, for example, vascular catheters, epidural catheters, endotracheal tubes, and urinary catheters. Nanocomposites, membranes, films, sandwiches, tubes, and the like, are encompassed by the present disclosure (see, e.g., Fong, et al. (2010) Acta. Biomater. 6:2554-2556; Huynh, et al (2010) Eur. J. Pharm. Biopharm. 74:255-264; Berra, et al (2008) Intensive Care Med. 34:1020-1029).

In embodiments, the disclosure encompasses methods for bulk distribution, gradient distribution, and limited surface distribution. Methods for manufacturing medical devices where an agent such as chlorhexidine is bulk distributed, gradient distributed, or limited surface distributed, are available (see, e.g., U.S. Pat. Nos.4,925,668 issued to Khan, et al, U.S. Pat. No. 5,165,952 issued to Solomon and Byron, and U.S. Pat. No. 5,707,366 issued to Solomon and Byron). In some aspects, the disclosed device excludes embodiments with bulk distribution.

The following terminology is for use in describing the concentration of any agent, for example, an anti-microbial agent, in a medical device, such as a catheter, or a related composition. The medical device has an external surface portion, and an internal volume portion, where a representational part of the internal volume comprises an area of the external surface portion. This representational part of the internal volume, in some embodiments, extends about 10 micrometers (um) down from the external surface into the interior, extends about 20um, extends about 40um, extends about 60um, extends about 80um, extends about 100um, extends about 120um, extends about 140um, extends about 160um, extends about 180um, extends about 200um, extends about 300um, extends about 400um, extends about 600um, extends about 800um, extends about 1000um (1.0mm), and the like. A selected representational part of the internal volume, for example, when sampled from the outer surface of a catheter or from an internal lumen of a catheter, contains the agent at a concentration of at least 5 micromolar (5uM), at least 10uM, at least 20uM, at least 40uM, at least 60uM, at least 80uM, at least 100uM, at least 120uM, at least 140uM, at least 160uM, at least 180uM, at least 200uM, at least 300uM, at least 400uM, at least 600uM, at least 800uM, at least 1000uM (1.0mM), at least 2mM, at least 5mM, at least 10mM, at least 15mM, at least 20mM, at least 25mM, at least 30mM, at least 40mM, at least 60mM, at least 80mM, at least 100mM, at least 150mM, at least 200mM, at least 250mM, and the like. In this context, the concentration unit of molarity is a surrogate for concentration of moles of agent per 100 cubic centimeters (one liter) of the selected internal volume of the medical device.

The disclosure encompasses a medical device treated with one or more of the presently described formulations, where the formulation contains chlorhexidine. For measurement, representative sample can be acquired by way of a sample that has a cubical conformation, a rectangular conformation, a cylindrical conformation, an amorphous conformation, as long as the sample is believed to be representative of the distribution (or concentration) of the agent in the region between the external surface and selected depth, or in a deeper region, for example, in a region between 50 micrometers deep and 200 micrometers deep.

Where chlorhexidine binds only to the surface of a medical device, such as a catheter, documentation of data on coating may be more meaningfully expressed in terms of micrograms chlorhexidine per square millimeter (and less meaningfully expressed in terms of micrograms chlorhexidine per cubic millimeter). The agent of the present disclosure is not limited to small molecules or to antimicrobials. What is encompassed is any agent of clinical use, or any agent that enhances one or more properties of the medical device, where the agent is substantially or completely soluble in the formulation. Thus, the agent can be a polymer with antimicrobial properties, where the polymer is substantially or completely soluble in the formulation.

The concentration can also be measured in situ, for example, with a technique involving fluorescence, radioactivity, or microbiological assays. Catheter is a non-limiting example. A microbiological assay configured for measuring the concentration of the amount of antimicrobial within a catheter can be measured as follows. A series of catheters, pre-impregnated with various concentrations of known anti-microbial, can be inoculated with the same quantity of a bacterium. The inoculated catheter can then be incubated under conditions suitable for growth of the bacteria, for example, including nutrients and a temperature of 37 degrees C. Following an incubation time of, for example, 1-7 days, the quantity of bacterial can then be measured. The amount of impregnated antimicrobial can be expressed in terms of a unit of percent maximal efficacy, or the amount of impregnated antimicrobial can be expressed with reference to a standard catheter containing a known quantity of antimicrobial. Methods are available for converting any organic molecule, such as chlorhexidine, into a corresponding radioactive molecule that contains tritium.

The present disclosure provides a formula that, when impregnated into a medical device, and when tested in the above microbiological assay, results in less than 80% maximal number of bacteria, less than 60%, less than 40%, less than 20%, less than 10%, less than 10%, less than 5%, less than 1%, less than 0.1%, less than 0.01%, less than 0.001%, less than 0.0001%, maximal number of bacteria. Maximal number of bacteria is measured with a control medical device, where the control medical device had been treated with solvents only (but not with any antimicrobial agent).

In some embodiments of the microbiological assay, the culturing medium is a complete nutrient medium that allows growth of the test organism. In other embodiments, the culturing medium is an incomplete nutrient medium that allows maintenance of the test organism, but does not support growth.

In embodiments that exclude, the present disclosure excludes a medical device or related composition, where the concentration is less than 5 micromolar (5uM), less than 10uM, less than 20uM, less than 40uM, less than 60uM, less than 80uM, less than 100uM, less than 120uM, less than 140uM, less than 160uM, less than 180uM, less than 200uM, less than 300uM, less than 400uM, less than 600uM, less than 800uM, less than 1000uM (1.0mM), less than 2mM, less than 5mM, less than 10mM, less than 15mM, less than 20mM, less than 25mM, less than 30mM, less than 40mM, less than 60mM, less than 80mM, less than 100mM, and so on.

The hardness of the devices of the present disclosure, including hardness of specific features, such as a tip, wall, bump, tapered region, hub, wing, tab, conical region, bead-like region, can be measured by the durometer method and Shore hardness scale. See, e.g., U.S. Pat. No. 5,489,269 issued to Aldrich and Cowan, U.S. Pat. No. 7,655,021 issued to Brasington and Madden, and Eleni, et al. (2011) Effects of outdoor weathering on facial prosthetic elastomers. Odontology. 99:68-76.

The present disclosure encompasses Shore A embodiments and Shore D embodiments. For example, a catheter, an internal lumen coating, an external coating, and such, can have (or can provide) a durometer value of about 40 to about 80 on a Shore A scale, about 45 to about 75 on a Shore A scale, about 50 to about 70 on a Shore A scale, about 55 to about 65 on a Shore A scale, or with a value of at least 10, at least 20, at least 30, at least 40, at least 50, at least 60, at least 70, at least 80, at least 90, at least 100, at least 120, at least 140, and the like, on a Shore A scale. Moreover, the dilator, a specific region or component of the dilator, or other device, such as a sheath, can have a value of less than 10, less than 20, less than 30, less than 40, less than 50, less than 60, less than 70, less than 80, less than 90, less than 100, less than 120, less than 140, and the like, on a Shore A scale. In other hardness embodiments, the disclosure provides a device (or a coating) with a durometer value of about 40 to about 80 on a Shore D scale, about 45 to about 75 on a Shore D scale, about 50 to about 70 on a Shore D scale, about 55 to about 65 on a Shore D scale, or with a value of at least 10, at least 20, at least 30, at least 40, at least 50, at least 60, at least 70, at least 80, at least 90, at least 100, at least 120, at least 140, and the like, on a Shore D scale. Moreover, the catheter, internal coating, or external coating, can have a value of less than 10, less than 20, less than 30, less than 40, less than 50, less than 60, less than 70, less than 80, less than 90, less than 100, less than 120, less than 140, and the like, on a Shore D scale.

At a given concentration of polymer in solution, where the polymer in solution is a component of a given formulation, the hardness value of the polymer can be chosen so that the solution of chosen polymer has a viscosity that is greater than that of a solution of a comparator polymer. In embodiments, the solution of chosen polymer has a viscosity that is at least 5% greater, at least 10%, at least 15%, at least 20%, at least 30%, at least 40%, at least 50%, at least 60%, at least 80%, at least 100% (twice as great), at least 1.5-fold, at least 2.0-fold, at least 4.0-fold, at least 6.0-fold, at least 8.0-fold, at least 10-fold, and the like, greater than that with comparator polymer.

At a given concentration of polymer in solution, where the polymer in solution is a component of a given formulation, the hardness value of the polymer can be chosen so that the solution of chosen polymer provides a mechanical adherence of the coating to the medical device body that is greater than that of a solution of a comparator polymer. Without implying any limitation, mechanical adherence of the coating can be measured by subjecting coated medical device to a number of flexing cycles, e.g., 1,000 cycles, 5,000, 10,000, 15,000, 50,000, 100,000, 150,000, 500,000 cycles, and the like. In embodiments, the solution of chosen polymer provides a mechanical adherence of coating to medical device body that is greater than that with comparator polymer, where mechanical adherence is at least 5% greater, 10%, 20%, 40%, 60%, 80%, 100% (twice that), 4-fold, 6-fold, 8-fold, 10-fold, at least 20-fold greater, and the like.

At a given concentration of polymer in solution, where the polymer in solution is a component of a given formulation, the hardness value of the polymer (or the concentration of the polymer in solution) can be chosen so that the solution of chosen polymer slows down release of chlorhexidine from the medical device. The slowing of release can be relative to a medical device coated with a comparator polymer (the comparator polymer can have a different hardness value). Alternatively, the slowing of release can be relative to a medical device, coated with the same polymer but at a lesser concentration. The viscosity of the solution that contains soluble polymer, can result in a coated medical device, where chlorhexidine release is less than 100% maximal rate of release, less than 95%, less than 90%, less than 80%, less than 70%, less than 50%, less than 20%, and the like.

The viscosity of solutions and formulations, including those comprising polyurethane can be measured using available instruments and methods. See, for example U.S. Pat. No. 8,017,686 issued to Buter, et al, and U.S. Pat. No. 5,091 ,205 issued to Fan. The Brookfield viscometer is a standard instrument (Brookfield Engineering Laboratories, Middleboro, MA). Equipment and methods for burst tests are available. See, e.g., Uson Testra static burst tester; Uson, Houston, Texas. The burst test can be destructive or non-destructive.

Thermoplastic polyurethane (TPU) tubing, resins, and the like, are available for use in the present disclosure, for example, as a medical device such as a catheter, as a coating for the medical device, as a formula configured for use in coating the medical device, or as a medical device that is modified by coating with the formula. What is available is tubing, resins, and the like, having a hardness of 72A, 77A, 87A, 94A, 51D, 60D, 63D, 67D, 73A/78A, 83A/86A, 90A/95A, 93A/98A, 55D/65D, 63D/78D, 73D, 75D/82D (Tecoflex® series); and 75A, 85A, 94A, 54D, 64D, 69D, 74D, 75D, 77A/83A, 87A/88A, 97A/97A, 55D/64D, 67D/75D, 70D, 75D, 77D/84D (Tecothane® series) (Lubrizol's Engineered Polymers for Medical and Health Care; Lubrizol Corp, Cleveland OH). Guidance on medical polymers, including polyurethane, is available, for example, from Polymer Membranes/Biomembranes (Advances in Polymer Science), ed. by Meier and Knoll, Springer, 2009; Lubricating Polymer Surfaces by Uyama, CRC Press, 1998; and Polymer Grafting and Crosslinking, ed. by Bhattacharya, et al, Wiley, 2008.

Reagents, including high purity solvents, as well as polymer resins such as 95A resin, can be acquired from Lubrizol Corp., Cleveland, OH; Microspec Corp., Peterborough, NH; Polaris Polymers, Avon Lake, OH; U.S. Plastic Corp., Lima, OH; Sigma-Aldrich, St. Louis, MO; E.I. du Pont de Nemours and Company, Wilmington, DE; Dow Chemical Co., Midland, Ml. Polyurethane of durometer 95A is disclosed, for example, by US 2010/0082097 of Rosenblatt, et al, U.S. Pat. No. 6,517,548 issued to Lorentzen Cornelius, et al, and by U.S. Pat. No. 2011/0054581 of Desai and Reddy.

An anti-microbially effective quantity of an anti-microbial agent can be measured by a number of non-limiting methods. The agent can be solubilized in water or other aqueous solution, solubilized in a solvent such as dimethylsulfoxide (DMSO) and then dispersed into an aqueous solution, dispersed in an aqueous solution with sonication, or dispersed into an aqueous solution by associating with albumin. Where the anti-microbial agent resides in the surface of, or has been impregnated into, or has been bulk incorporated into, a medical device, the agent can be extracted from the device using a solvent (e.g., water, methanol, tetrahydrofuran, DMSO, and the like), or crushed or pulverized, and then extracted with solvent. Then, the solubilized or extracted anti-microbial can be tested for anti-microbially effective activity using chemical methods, e.g., high pressure liquid chromatography (HPLC) or microbiological assays, e.g., in solution or agar-based, using methods well known by the skilled artisan. Alternatively, anti-microbial efficacy of the medical device can be assessed by inoculating the medical device with a microbe, and by monitoring the ability of the antimicrobial agent to reduce growth, to reduce attachment, or to kill, the microbe. Anti-microbial activities taking place on the surface of the medical device, or within the matrix or pores of the medical device, can be assessed by light microscopy or electron microscopy, using methods well known to the skilled artisan. A medical device containing an anti-microbially effective amount of an anti-microbial agent can be measured by detecting the number of microorganisms that colonize the surface of a medical device or that colonize pores or a matrix of a medical device. Alternatively, and without limitation, anti-microbially effective can be measured by incubating the medical device in a liquid medium, or an agar medium, and by detecting the number of microorganisms that colonize the surface of medical device, or that colonize a pre-determined area or volume apart from the surface of the medical device, for example, an area that is 0mm to 1 mm away from the surface of the medical device, that is 1 mm to 3mm away, from 0mm to 3 mm away, 2mm to 5mm away, from 0mm to 5mm away, from 2mm to 20mm away, and the like. Control medical devices can be treated with sham formulation only (no anti-microbial) or can be treated with an active control.

Methods and equipment are available to the skilled artisan for measuring structures, properties, and functions, of medical devices, such as catheters. The following references disclose methods and equipment for measuring, for example, tensile strength, force at break, elastic behavior, plastic behavior, microscopy for detecting microbial colonies or biofilms residing on the surface of catheters, microbiological assays for measuring influence of anti-microbials. See, e.g., Aslam and Darouiche (2010) Infect. Control Hosp. Epidemiol. 31:1 124-1 129; Hachem etal (2009) Antimicrobial Agents Chemotherapy 53:5145-5149; Venkatesh et al (2009) J. Medical Microbiol. 58:936-944. Methods and equipment for measuring tensile strength, elongation at break, and other properties of medical devices, are available. See, e.g., U.S. Pat. No. 6,039,755 issued to Edwin et al, and U.S. 7,803,395 issued to Datta et al. Above a limiting stress, called the elastic limit, some of the strain is permanent. In going beyond the elastic limit, a solid can either fracture suddenly or deform in a permanent way (see, e.g., Ashby MF, Jones DRH (2012) Engineering Materials 1, 4th ed., Elsevier, New York, pp. 1 15-133).

### Examples

### Internal formulation

Formulations and methods for preparing the internal solution are disclosed, as follows. Formulation of internal solution is shown (Table 1).

| Table 1. Internal solution. | |
|---|---|
| Methyl-ethyl-ketone | about 2000 grams |
| Methanol | about 400-500 grams |
| Acetone | about 600-700 grams |
| Chlorhexidine diacetate | about 50 grams |
| Chlorhexidine free base | about 50 grams |

The reagents are chlorhexidine base, chlorhexidine diacetate, methyl-ethyl-ketone (MEK), methanol (ACS grade), and acetone. As a general statement, without intending any limitation, methanol can prevent precipitation of chlorhexidine to a greater extent than certain other solvents.

### Elution studies of internally coating of internally coated catheter

For studies of elution of material from the internal coating of the dipped catheter, elution of material such as chlorhexidine was measured by soaking the catheter in citrated plasma.

### Formulation pH, precipitation of chlorhexidine, and chlorhexidine content

Readings of pH, for various formulations, were conducted shortly after a test strip was wetted with a coating solution. A "dry" reading was recorded after the test strip had completed a drying cycle. Wet and "dry" readings for various formulations were as follows. The trivial names of the formulations are MAR091, MAR092, MAR093, and MAR094. The pH readings were MAR091 (wet pH 7, dry pH 10), MAR092 (wet pH 7, dry pH 8), MAR093 (wet pH 6, dry pH 6), and MAR094 (wet pH 6, dry pH 6). Related work demonstrated that solutions with alkaline pH values let to precipitation of chlorhexidine. The formulations included the following amounts (%) of MEK, methanol, acetone, CHA, and CHX, respectively. MAR091 (65%; 30%; 0%; 50%; 50%). MAR091 also included 5% acetonitrile. MAR092 (65%; 30%; 0%; 50%; 50%). MAR092 also included 5% THF. MAR093 (65%; 15%; 20%; 50%; 50%). MAR094 (65%; 20%; 15%; 50%; 50%). Chlorhexidine content, in terms of micrograms/cm, of treated catheters was measured. Treated catheters had the indicated chlorhexidine content: MAR091 (23 micrograms/cm), MAR092 (26 micrograms/cm), MAR093 (30 micrograms/cm), and MAR094 (29 micrograms/cm).

The disclosure provides one or more formulations, where the pH is less than 9.0, less than 8.5, less than 8.0, less than 7.5, less than 7.0, less than 6.5, or where the pH is between 5.0-9.0, between 5.0-8.5, between 5.0-8.0, between 5.0-7.5, between 5.0-7.0, between 5.0-6.5, between 5.0-6.0, and the like. Formulation can be applied to a commercially available, e.g., pH indicator paper, pH test strips, or pH indicator strips from Sigma Aldrich, St. Louis, MO. Moisture present in the formulation and/or in the pH paper is sufficient to obtain a pH reading of formation. The skilled artisan can acquire pH value of a formulation that is dried on a substrate, or a pH value of a formulation that is non-aqueous, by adding distilled water, e.g., 0.05mL, 0.10mL, 0.20mL, 0.5mL, 1.0mL, of neutral, buffer-free distilled water, and by dissolving the formulation in the distilled water.

### External formulation

Formulation and method for preparing external solution is disclosed, as follows. Formulation is disclosed (Table 2).

| Table 2. External formulation | |
|---|---|
| Tetrahydrofuran (THF) | about 2000-2500 grams |
| Methanol | about 200-300 grams |
| Polyurethane 95A | about 100-200 grams |
| Chlorhexidine diacetate | about 50 grams |

### Elution studies of external coating of externally coated catheter

For studies of elution of material from the external coating of the dipped catheter, elution of material such as chlorhexidine was measured by soaking the catheter in normal saline.

### Assay method for chlorhexidine

Chlorhexidine was extracted form samples of catheters, or of other medical devices. Analysis used high pressure liquid chromatography (HPLC) using a column (Agilent, Santa Clara, CA). Detection of chlorhexidine was with light at 280 nm. The method was standardized using known standards of chlorhexidine (75.0 micrograms/mL).

### Measuring chlorhexidine content of treated catheters

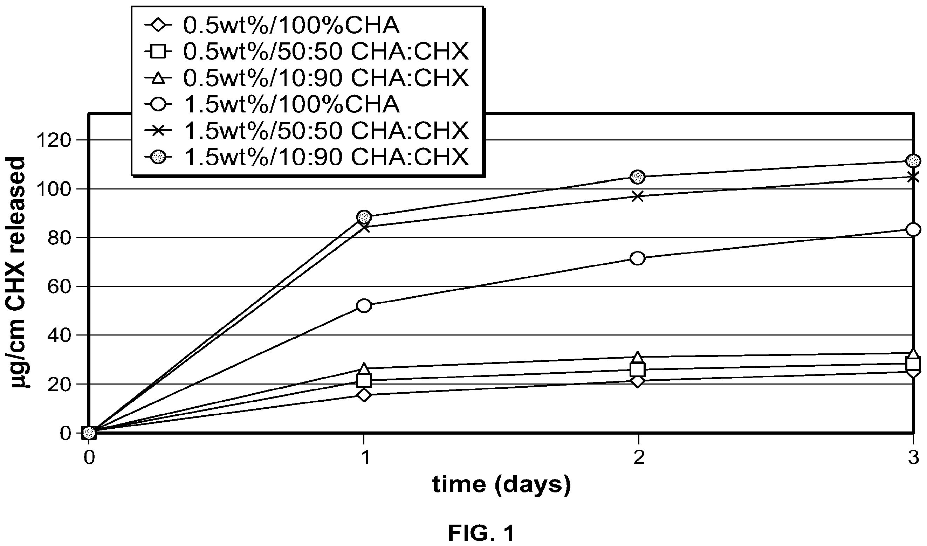

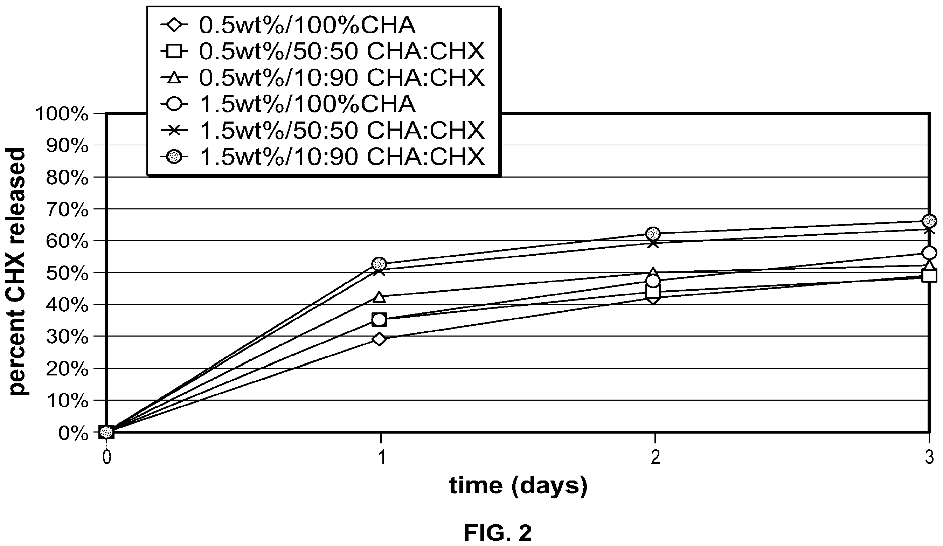

Table 3 discloses the chlorhexidine content (micrograms/cm) where total chlorhexidine in the treating solution was 0.5 wt.% or 1.5 wt.%, as indicated, and where chlorhexidine takes the form of 100% chlorhexidine diacetate (CHA), or where the chlorhexidine takes the form of 50/50 chlorhexidine diacetate (CHA)/chlorhexine base (CHX), as indicated. The following concerns the content of chlorhexidine in the treated catheters, prior to conducting time-course elution experiments. As shown in Table 3, as the CHA was increased (in the ratio of CHA to CHX in the treatment solution) the content on the catheter slightly decreased. The slight drop in content observed when comparing the 50/50 CHA/CHX solutions and 100% CHA solutions is attributed to the fact that a portion of the weight of CHA (about 20%) is acetate, whereas the weight of CHX is pure chlorhexidine. Table 3 discloses the concentrations at t = zero days, as it applies to the 3-day time course experiments shown in Fig. 1 and Fig. 2.

| Table 3. Chlorhexidine content of treated catheters. | | | | |
|---|---|---|---|---|
| Trivial name of solution | Solution for treating catheter each with polyurethane resin | | | Chlorhexidine content (micrograms/cm) |
| JAN031 | 0.5 wt % | 50/50 CHA/CHX | 85% THF, 15% methanol | 58.20 |
| JAN030 | 0.5 wt % | 100% CHA | 85% THF, 15% methanol | 51.17 |
| JAN032 | 0.5 wt % | 10/90 CHA/CHX | 85% THF, 15% methanol | 61.98 |
| JAN034 | 1.5 wt % | 50/50 CHA/CHX | 85% THF, 15% methanol | 164.85 |
| JAN033 | 1.5 wt % | 100% CHA | 85% THF, 15% methanol | 151.42 |
| JAN035 | 1.5 wt % | 10/90 CHA/CHX | 85% THF, 15% methanol | 169.06 |

Figure 1 reveals cumulative elution of chlorhexidine (micrograms/cm) over time, where treatment of catheters was with solutions of 0.5% or 1.5% chlorhexidine. Elution was followed for three days. The percentage refers to the total amount, by weight, of the chlorhexidine in the treatment solution. The solutions of chlorhexidine were 100% chlorhexidine diacetate (CHA), 50/50 chlorhexidine diacetate/chlorhexidine base (CHA/CHX), or 10/90 CHA/CHX. Lower rates of release were found with 0.5% wt% (diamonds, squares, triangles), as compared to data where catheters were treated with solutions of 1.5% (X (upper X curve), X (lower X curve), X (lower filled circles curve). The lowest rate of elution was where the coating procedure used 100% CHA, while the fastest rate of elution occurred where the coating procedure used 10/90 CHA/CHX. Thus, where the goal is to acquire a medical device with prolonged time-release, treatment solutions with 100% CHA is preferred.

Figure 2 illustrates cumulative elution of chlorhexidine (percent release) over time, where treatment of catheters was with 0.5% or 1.5% chlorhexidine. Elution was monitored for three days. The lowest rate of elution was where the coating procedure used 100% CHA, while the fastest rate of elution occurred where the coating procedure used 10/90 CHA/CHX.

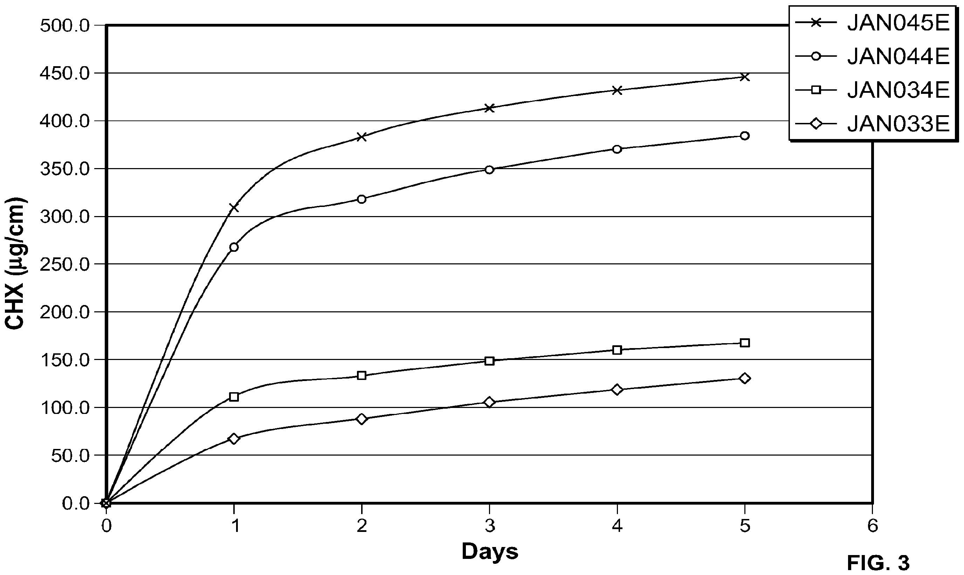

Figure 3 demonstrates cumulative elution of chlorhexidine (micrograms/cm), where treatment of catheters was with 1.5% or 3.0% chlorhexidine. Elution was followed for five days. Table 4 lists the treatment solutions, and the initial chlorhexidine content of the catheters with the four treatments. This represents a different set of treated catheters than the set represented by Table 3. Table 4 shows the concentration at t = zero days, in the 5-day time course experiments shown in Fig. 3, Fig. 4, and Fig. 5. Where the treatment solution contained 100% CHA, the rate of elution was slower, as compared to elution where the treatment solution contained 50/50 CHA/CHX (Fig. 3). The slower elution with the 100% CHA catheters was found where treatment was with the lower total concentration. Where the higher total concentration (3.0%) of chlorhexidine was used in the treatment solution, treatments with 100% CHA resulted in lower rates of elution, during the time-course test (Fig. 3).

To summarize, slower rates of elution were found with 3 percent of 100% CHA (X-points), as compared with 3 percent of 50/50 CHA/CHX (open square-points). Also, slower rates of elution were found with 1.5 percent 100% CHA (closed diamond-points), when compared with 1.5 percent 50/50 CHA/CHX (closed square-points). Thus, where the goal is to acquire a medical device with prolonged time-release, treatment solutions with 100% CHA is preferred.

Photographs of catheters treated with formulations JAN045, JAN034, JAN044, AND JAN033 were taken. The photographs disclose shark skin appearance, whiteness caused by flexing, tiny bubbles, small surface defects, and absence of defects.

In embodiments, what is provided is a medical device, e.g., catheter, cannula, or introducer, with chlorhexidine content of at least 150micrograms/cm, at least 175, at least 200, at least 225, at least 250, at least 275, at least 300, at least 325, at least 350, at least 375, at least 400, at least 425, at least 450, at least 475, at least 500, at least 525, at least 550, at least 575, at least 600, at least 625, at least 650, at least 675, at least 700, and the like, micrograms/cm. In exclusionary embodiments, the invention excludes a medical device where the chlorhexidine content (micrograms/cm) is less than 650, 625, 600, 575, 550, 525, 500, 475, 450, 425, 400, 375, 350, 325, 300, 275, 250, 225, 200, 175, 150, 125, 100, 75, 50, and the like, micrograms/cm.

| Table 4. Chlorhexidine content of treated catheters. | | | | |
|---|---|---|---|---|
| Trivial name of solution | Solution for treating catheter. All solutions contained polyurethane resin. | | | Chlorhexidine content (micrograms/cm) |
| JAN033 | 1.5 wt % | 100% CHA | 85% THF, 15% methanol | 200.5 |
| JAN034 | 1.5 wt % | 50/50 CHA/CHX | 85% THF, 15% methanol | 227.4 |
| JAN044 | 3.0 wt % | 100% CHA | 85% THF, 15% methanol | 430.5 |
| JAN045 | 3.0 wt % | 50/50 CHA/CHX | 85% THF, 15% methanol | 488.1 |

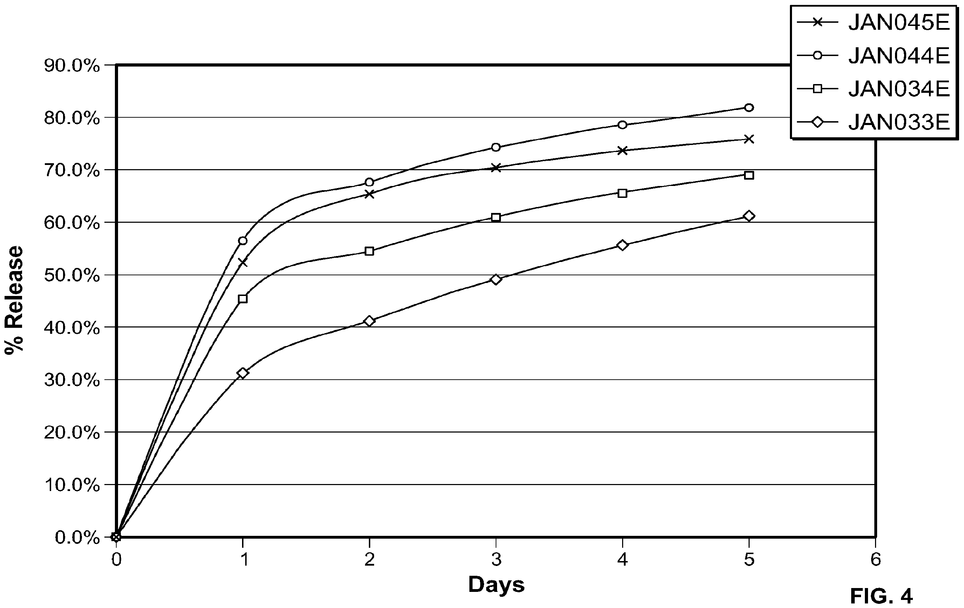

Figure 4. Cumulative elution of chlorhexidine (percent release) over time, where treatment of catheters was with 1.5% or 3.0% chlorhexidine. Elution was followed for five days. Table 2 discloses the treatment solutions and initial chlorhexidine content of the catheters, with each of the four treatments. Where the treatment solution contained 100% CHA, the rate of elution was slower, as compared to elution where the treatment solution contained 50/50 CHA/CHX (Fig. 4). The slower elution with the 100% CHA catheters was found where treatment was with the lower total concentration. But with where the treatment solution had the higher total concentration of chlorhexidine, the 100% CHA catheters showed a somewhat higher elution rate, as compared with the 50/50 CHA/CHX catheters. (Fig. 4).

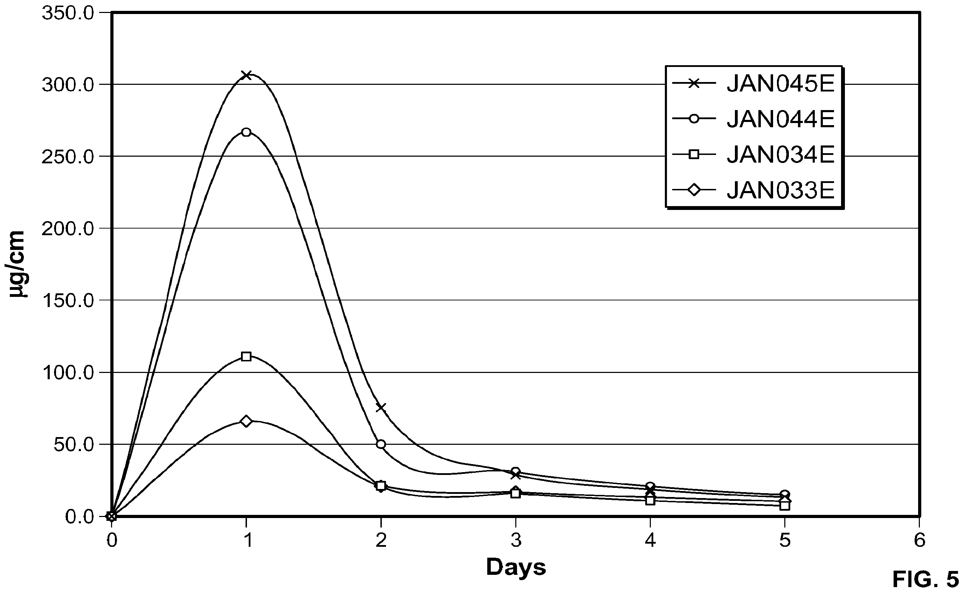

Figure 5 demonstrates the quantity of chlorhexidine eluted per day. In other words, the data presented represent results on a per day basis (not cumulative results). Table 4 lists the treatment solutions and initial chlorhexidine content of the catheters, with each of the four treatments. In all tests, where the treatment solution contained 100% CHA, elution occurred at a slower rate than where treatment solution contained 50/50 CHA/CHX.

### Surface characteristics of treated catheters

Catheters coated according to Table 4 were evaluated. Table 4 discloses four types of treatment solutions. With 3.0% chlorhexidine 50/50 CHA/CHX, the surface was rough and resembled that of shark skin. When the catheter was flexed, the area that was flexed turned white from stress whitening. The other catheters had a better appearance, though the 3.0 wt% chlorhexidine 100% CHA had small bubbles on the surface, and 1.5% chlorhexidine 100% CHA had small defects on the surface.

### Effect of different percentages of tetrahydrofuran and methanol in the treatment solutions

Changing the percentages of tetrahydrofuran (THF) and methanol, in treatment solutions, resulted in changes in various characteristics of the catheters, as measured after treatment. Treatment solutions containing 70% THF/30% methanol or 85% THF/15% methanol were tested. Table 5 identifies these non-limiting solutions.

| Table 5. Solutions for treating catheters. Each solution had polyurethane resin. | | | |
|---|---|---|---|
| Trivial name of solution | Total wt % of chlorhexidine | Ratio of CHA/CHX | Quantities of THF, methanol, and resin |
| FEB061 | 2.0 wt % | 50/50 CHA/CHX | 70% THF, 30% methanol |
| DEC028 | 2.0 wt % | 50/50 CHA/CHX | 85% THF, 15% methanol |

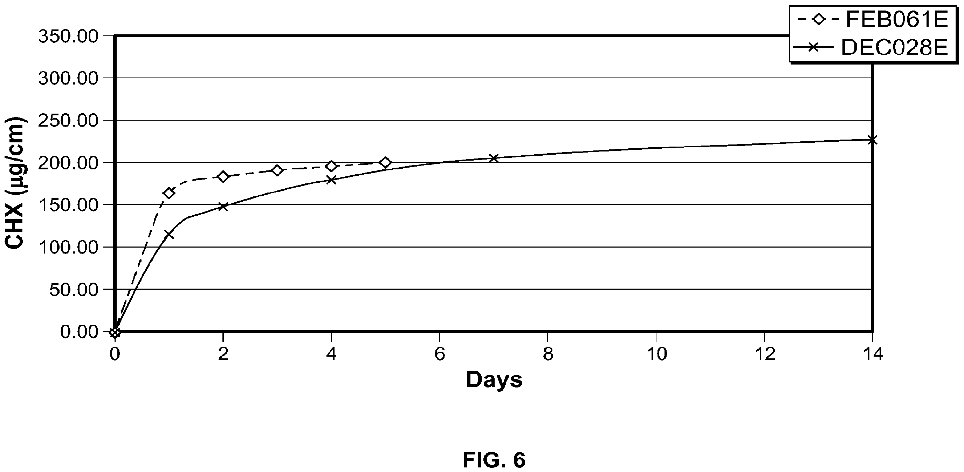

The treated catheters were subjected to time-course tests for elution of chlorhexidine. Figure 6 demonstrates that samples prepared with the lower THF solution had a higher initial release of chlorhexidine, while samples prepared with higher THF solution had lower initial release rate (Fig. 6). Thus, where the goal is to acquire a medical device with prolonged time-release, treatment solutions with a lower relative THF concentration is preferred.

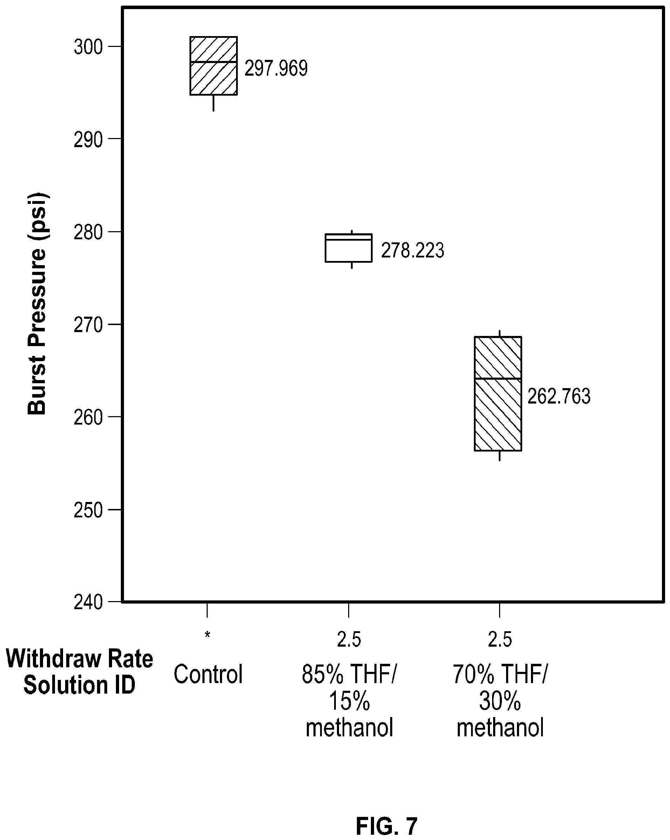

Figure 7 discloses results from burst pressure (psi) experiments of uncoated and coated catheters. Catheters were subject to no treatment (control), to treatment with higher THF solution, or to treatment with lower THF solution. The higher THF solution contained 85% THF, 15% methanol, overall chlorhexidine 2 wt.%, 50/50 CHA/CHX, and polyurethane resin. The lower THF solution contained 70% THF, 30% methanol, overall chlorhexidine 2 wt.%, 50/50 CHA/CHX, and polyurethane resin.

Figure 7 shows the burst pressure of uncoated and coated catheters. Burst pressure was lower with the 70% THF/30% methanol solution, and higher with the 85% THF/15% methanol solution. The drop in burst strength with lower THF content may have been due to the increase in methanol content, where the increased methanol provoked deterioration of the catheter. The results demonstrate that high THF or lower methanol are preferred for a more robust burst strength.

### Fibrin weight and intimal hyperplasia

The following discloses results with peripherally inserted central catheters (PICC) using a preferred formulation, and two control formulations for coating and/or impregnating. Without implying any limitation, an infection model can involve rabbits with a bacterial challenge to a catheter by inoculating the insertion site with about 1 mL of inoculum of *Staphylococcus aureus.* Catheters can be anchored to the skin with adhesive tape and sutures. Infiltration of lumen of blood vessel with neutrophils, macrophages, or other indicia of inflammation can be measured. Location of bacterial accumulation in wall of blood vessel can be detected and quantified. In one embodiment, the indwelling catheter can be maintained in rabbit for two weeks, three weeks, four weeks, and so on. In the weight measurements, the reported weight was a mixture of visible clot/thrombus and fibrin sheath. The weight was measured after removal from the catheter. Any thrombus formation was removed from the catheter surface and weight in gram units.

### Infection models

Regarding the infection model, two sheep studies were run and inserted with coated products and uncoated products (separate groups) in their jugular veins with tip placement in superior vena cava. At 31 day, they were sacrificed to harvest vessels and catheters to evaluate amount of thrombus on catheter external surfaces. Infection model included introduction of *S. aureus* at insertion site to initiate infection to evaluate the impact of thrombus accumulation on catheter surfaces in presence of infection. The non-infection model did not include this step.

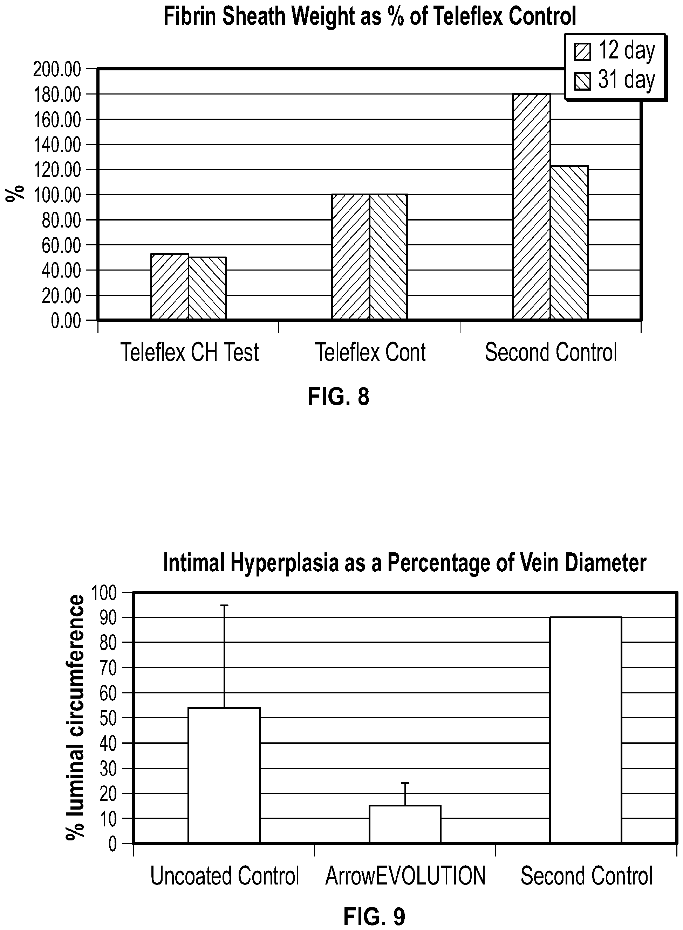

Figure 8 discloses fibrin sheath weight as percent of control, using a non-infection model. Compared to the two control catheters, catheters coated with preferred formulation showed the least increase of fibrin sheath weight, in the non-infection model.

Figure 9 discloses fibrin sheath weight as percent of control, using an infection model. Compared to the two control catheters, catheters coated with preferred formulation showed the least increase of fibrin sheath weight, in the infection model.

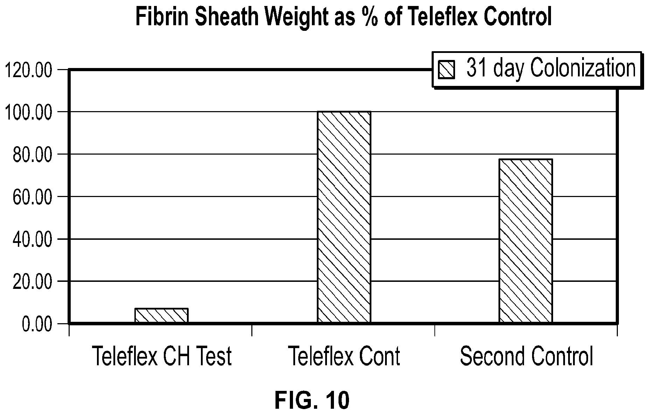

Figure 10 discloses results from a study of intimal hyperplasia as percentage of vein diameter. Compared to the two control catheters (coated control; uncoated control), catheters coated with preferred formulation showed the lowest value for intimal hyperplasia. The time from was 31 days of catheter indwelling, followed by harvesting of vessels. Intima thickness was measured on histology slides.

In embodiments, preferred formulations result in a reduction by at least 10%, by at least 15%, by at least 20%, by at least 30%, by at least 40%, by at least 50%, by at least 60%, by at least 70%, by at least 80%, by at least 90%, when compared to non-treated catheter, of one or more of fibrin content, increase in intimal thickness, inflammation (e.g., white blood cell count in intima), or thrombogenicity. In embodiments, preferred formulations result in a reduction by at least 10%, by at least 15%, by at least 20%, by at least 30%, by at least 40%, by at least 50%, by at least 60%, by at least 70%, by at least 80%, by at least 90%, when compared to catheter treated, coated, or impregnated, with alternate formulation, of one or more of fibrin content, increase in intimal thickness, inflammation (e.g., white blood cell count in intimal), or thrombogenicity.

## Claims

1. A medical device comprising an interior surface that defines a cavity or lumen, and an exterior surface, wherein the interior surface is treated with a first formulation including:
methyl-ethyl-ketone 50-70%; methanol 10-20%; acetone 15-25%; chlorhexidine diacetate 0.5-4%; and chlorhexidine free base 0.5-4% resulting in coating or impregnation with an antimicrobially effective amount of chlorhexidine, wherein the exterior surface of the medical device is treated with a second formulation including:
tetrahydrofuran (THF) 70-90% by weight; methanol 5-15%; polyurethane 1-15%; and chlorhexidine diacetate 0.5-4.0% resulting in coating or impregnation with an antimicrobially effect amount of chlorhexidine wherein the medical device does not comprise triclosan and does not comprise silver salt.

2. The medical device of Claim 1 that comprises one or more of a catheter, cannula, introducer, dilator, or sheath.

3. A method for coating or impregnating a medical device with an antimicrobially effective amount of chlorhexidine, the medical device comprising an inside surface, a cavity or lumen that is defined by the inside surface, and an outside surface, the method comprising:
contacting a first formulation to the inside surface, the first formulation including:
methyl ethyl ketone 50-70%; methanol 10-20%; acetone 15-25%; chlorhexidine diacetate 0.5-4%; and chlorhexidine free base 0.5-4% resulting in coating or impregnation with an antimicrobially effective amount of chlorhexidine; and
contacting a second formulation to the outside surface, the second formulation including:
tetrahydrofuran (THF) 70-90% by weight; methanol 5-15%; polyurethane 1-15%; and chlorhexidine diacetate 0.5-4.0% resulting in coating or impregnation with an antimicrobially effect amount of chlorhexidine, wherein the medical device does not comprise triclosan and does not comprise silver salt.

4. The medical device of Claim 1 configured to introduce fluids into a subject, to withdraw fluids from the subject, or to both introduce and withdraw fluids, wherein in operation the device is capable of dwelling in a physiological vessel or chamber, and is capable of introducing, withdrawing, or both introducing and withdrawing fluids to said physiological vessel or chamber, wherein in use the fluids are in contact with and transmitted by said cavity or lumen that is defined by said inside surface during the introducing and withdrawing.

5. The medical device of Claim 4, wherein the vessel is a vein.

## Patentansprüche

1. Medizinische Vorrichtung, die eine innere Oberfläche, die einen Hohlraum oder ein Lumen definiert, und eine äußeren Oberfläche umfasst, wobei die innere Oberfläche mit einer ersten Formulierung behandelt worden ist, die beinhaltet:
Methyl-ethyl-keton 50-70%; Methanol 10-20%; Aceton 15-25%; Chlorhexidindiacetat 0,5-4% und Chlorhexidin freie Base 0,5-4%, was zu einer Beschichtung oder Imprägnierung mit einer antimikrobiell wirksamen Menge an Chlorhexidin führt, wobei die äußere Oberfläche der medizinischen Vorrichtung mit einer zweiten Formulierung behandelt wird, die beinhaltet:
Tetrahydrofuran (THF) 70-90 Gew.-%; Methanol 5-15%; Polyurethan 1-15% und Chlorhexidindiacetat 0,5-4,0%, was zu einer Beschichtung oder Imprägnierung mit einer antimikrobiell wirksamen Menge Chlorhexidin führt, wobei die medizinische Vorrichtung kein Triclosan umfasst und kein Silbersalz umfasst.

2. Medizinische Vorrichtung nach Anspruch 1, die einen oder mehrere von einem Katheter, einer Kanüle, einer Einführhilfe, einem Dilatator oder einer Hülle umfasst.

3. Verfahren zum Beschichten oder Imprägnieren einer medizinischen Vorrichtung mit einer antimikrobiell wirksamen Menge Chlorhexidin, wobei die medizinische Vorrichtung eine innere Oberfläche, einen Hohlraum oder ein Lumen, das durch die innere Oberfläche definiert ist, und eine äußere Oberfläche umfasst, wobei das Verfahren umfasst:
Kontaktieren einer ersten Formulierung mit der inneren Oberfläche, wobei die erste Formulierung beinhaltet:
Methylethylketon 50-70%; Methanol 10-20%; Aceton 15-25%; Chlorhexidindiacetat 0,5-4% und Chlorhexidin freie Base 0,5-4%, was zu einer Beschichtung oder Imprägnierung mit einer antimikrobiell wirksamen Menge Chlorhexidin führt; und
Kontaktieren einer zweiten Formulierung mit der äußeren Oberfläche, wobei die zweite Formulierung beinhaltet:
Tetrahydrofuran (THF) 70-90 Gew.-%; Methanol 5-15%; Polyurethan 1-15% und Chlorhexidindiacetat 0,5-4,0%, was zu einer Beschichtung oder Imprägnierung mit einer antimikrobiell wirksamen Menge an Chlorhexidin führt, wobei die medizinische Vorrichtung kein Triclosan umfasst und kein Silbersalz umfasst.

4. Medizinische Vorrichtung nach Anspruch 1, die so konfiguriert ist, dass sie Fluide in ein Subjekt einführt, Fluide aus dem Subjekt herauszieht oder Fluide sowohl einführt als auch herauszieht, wobei die Vorrichtung im Betrieb in der Lage ist, in einem physiologischen Gefäß oder einer physiologischen Kammer zu verweilen und in der Lage ist, Fluide in das physiologische Gefäß oder die physiologische Kammer einzuführen, herauszuziehen oder sowohl einzuführen als auch herauszuziehen, wobei die Fluide bei Gebrauch während des Einführens und Herausziehens in Kontakt stehen mit und übertragen werden durch den durch die innere Oberfläche definierten Hohlraum oder Lumen.

5. Medizinische Vorrichtung nach Anspruch 4, wobei das Gefäß eine Vene ist.

## Revendications

1. Dispositif médical comprenant une surface intérieure qui définit une cavité ou une lumière et une surface extérieure, dans lequel la surface intérieure est traitée avec une première formulation incluant :
50-70 % de méthyl-éthyl-cétone ; 10- 20 % de méthanol ; 15-25 % d'acétone ; 0,5-4 % de diacétate de chlorhexidine ; et 0,5-4% de base libre de chlorhexidine résultant en un revêtement ou une imprégnation avec une quantité antimicrobienne efficace de chlorhexidine, dans lequel la surface extérieure du dispositif médical est traitée avec une seconde formulation incluant :
70-90 % en poids de tétrahydrofurane (THF) ; 5-15 % de méthanol ; 1-15 % de polyuréthane ; et 0,5-4,0 % de diacétate de chlorhexidine résultant en un revêtement ou une imprégnation avec une quantité antimicrobienne efficace de chlorhexidine, dans lequel le dispositif médical ne comprend pas de triclosan et ne comprend pas de sel d'argent.

2. Dispositif médical selon la revendication 1, qui comprend un ou plusieurs parmi un cathéter, une canule, un introducteur, un dilatateur ou une gaine.

3. Procédé pour revêtir ou imprégner un dispositif médical avec une quantité antimicrobienne efficace de chlorhexidine, le dispositif médical comprenant une surface intérieure, une cavité ou une lumière qui est définie par la surface intérieure et une surface extérieure, le procédé comprenant :
mettre en contact une première formulation avec la surface intérieure, la première formulation incluant :
50-70 % de méthyl-éthyl-cétone ; 10- 20 % de méthanol ; 15-25 % d'acétone ; 0,5-4 % de diacétate de chlorhexidine ; et 0,5-4 % de base libre de chlorhexidine résultant en un revêtement ou une imprégnation avec une quantité antimicrobienne efficace de chlorhexidine, et
mettre en contact une seconde formulation avec la surface extérieure, la seconde formulation incluant :
70-90 % en poids de tétrahydrofurane (THF) ; 5-15 % de méthanol ; 1-15 % de polyuréthane ; et 0,5-4,0 % de diacétate de chlorhexidine résultant en un revêtement ou une imprégnation avec une quantité antimicrobienne efficace de chlorhexidine, dans lequel le dispositif médical ne comprend pas de triclosan et ne comprend pas de sel d'argent.

4. Dispositif médical selon la revendication 1 configuré pour introduire des fluides dans un sujet, pour retirer des fluides du sujet, ou à la fois pour introduire et retirer des fluides, dans lequel en fonctionnement le dispositif est capable de loger dans un vaisseau ou une chambre physiologique et est capable d'introduction, de retrait ou à la fois d'introduction et de retrait de fluides dans ou à partir du vaisseau ou chambre physiologique, dans lequel en fonctionnement les fluides sont en contact avec et transmis par ladite cavité ou lumière qui est définie par ladite surface intérieure lors de l'introduction et du retrait.

5. Dispositif médical selon la revendication 4, dans lequel le vaisseau est une veine.
